# EUROPÄISCHE PATENTANMELDUNG

(11) **EP 0 808 817 A1**
(43) Veröffentlichungstag der Anmeldung: **26.11.1997**
(21) Anmeldenummer: 97107716.9
(22) Anmeldetag: 12.05.1997
(51) Int. Cl.: C07C 63/68, C07C 51/363

(54) **Verfahren zur Herstellung von halogenierten Phthalsäuren und deren Derivaten**

(30) Priorität: 23.05.1996 DE 19620797
(71) Anmelder: BAYER AG, 51368 Leverkusen (DE)
(72) Erfinder: Pelster, Thomas, Dr., 50999 Köln (DE); Birmes, Hartmut, Dr., 42327 Wuppertal (DE); Michaelis, Stephan, Dr., 51519 Odenthal (DE)

(57) **Zusammenfassung**

Verfahren zur Herstellung halogenierter Phthalsäuren bzw. deren Derivate durch Halogenierung von Phthalsäuren bzw. deren Derivate mit einem Halogenierungsmittel in rauchender Schwefelsäure in Gegenwart einer iodhaltigen Verbindung als Katalysator, dadurch gekennzeichnet, daß die Halogenierung bei einer Temperatur von 100 bis 160°C erfolgt.

## Beschreibung

Die Erfindung betrifft ein Verfahren zur Herstellung von halogenierten Phthalsäuren und deren Derivaten sowie deren Verwendung.

Halogenierte Phthalsäuren bzw. deren Derivate, wie Anhydride, insbesondere Tetrachlor- und Tetrabromphthalsäure-Anhydride sind wichtige Zwischenprodukte für die Herstellung von Farbstoffen, Pigmenten, Fungiziden, Polyester- und Epoxidharzen mit flammhemmenden Eigenschaften sowie als flammhemmende Zusätze in Anstrichmitteln.

Die Herstellung von Tetrachlorphthalsäure bzw. -Anhydrid erfolgt beispielsweise gemäß dem in US-A-2 460 564 beschriebenen Verfahren durch Chlorierung von Phthalsäureanhydrid in der Schmelze und in Gegenwart von Fe(III)Cl₃ oder Molybdänchlorid, wobei eine Temperatur von bis zu 270°C erforderlich ist.

Gemäß dem in Chem. Abstract 61 (1964) 11927, beschriebenen Verfahren wird Phthalsäureanhydrid bei mindestens 270°C in der Gasphase in Gegenwart von Fe(III)Cl₃ mit Cl₂ umgesetzt.

Gemäß CH-A-50 177 erfolgt die Herstellung von halogenierten Phthalsäureanhydriden wie Tetrachlor-, Tetrabom- oder Tetraiodphthalsäureanhydrid jeweils ausgehend von Phthalsäureanhydrid in rauchender Schwefelsäure, die einen SO₃-Gehalt von 50 bis 60 Gew.-% besitzt, bei einer Temperatur von 180 bis 200°C.

Diese Verfahren liefern aus verschiedenen Gründen keine zufriedenstellende Ergebnisse, da sie beispielsweise ein Arbeiten bei hohen Temperaturen erforderlich machen, wobei große Mengen an Verunreinigungen entstehen. Zudem sind so hergestellte halogenierte Phthalsäuren bzw. deren Anhydride meist dunkel gefärbt. Zur Reinigung solch dunkel gefärbter Verfahrensprodukte wurde gemäß US-A-2 547 504 der Herstellung ein Reinigungsschritt nachgeschaltet, bei dem das Produkt in Gegenwart von alkalischen Salzen auf Temperaturen oberhalb von 260°C erhitzt und anschließend destilliert wird. Zusätzliche Verfahrensschritte sind jedoch kostenintensiv, bringen - wie hier - Ausbeute-Verlust und sollten daher möglichst vermieden werden.

In DE-A-1 934 174 wird zwar ein Verfahren beschrieben, gemäß dem eine nachgeschaltete Reinigung des Verfahrensproduktes nicht mehr nötig sein soll. Dabei wird Chlorsulfonsäure als ausschließliches Lösungsmittel verwendet. Dieses Verfahren besitzt beispielsweise den Nachteil, daß bei einer wäßrigen Aufarbeitung nicht nur die bei der Zersetzung von Chlorsulfonsäure entstehende HCl, sondern auch die bei der Chlorierung freigesetzte HCl entsorgt werden müssen.

Gemäß dem in EP-A 632 032 beschriebenen Verfahren wird Tetrachlorphthalsäureanhydrid wie es nach einzelnen Herstellungsverfahren als Rohprodukt anfällt gereinigt. Als zu entfernende Nebenprodukte in derartigen Rohprodukten wird u.a. Iodtrichlorphthalsäure beschrieben, deren Entfernung über eine Radikalreaktion, insbesondere durch Bestrahlung mit kurzwelligem UV-Licht in chlorierten Lösungsmitteln realisiert wird. Für die Entfernung anderer Verunreinigungen werden anderere Reiningungsschritte angegeben. Auch hier ist wiederum die aufwendige bzw. kostenintensive Aufeinanderfolge von Verfahrensschritten nachteilig.

Aufgabe der Erfindung war es daher, ein Verfahren zur Herstellung von halogenierten Phthalsäuren bzw. deren Derivaten bereitzustellen, das zum einen die oben beschriebenen Nachteile nicht besitzt und bei dem zum anderen so gut wie keine Verunreinigungen mehr entstehen.

Es wurde nun ein Verfahren zur Herstellung von halogenierten Phthalsäuren bzw. deren Derivaten durch Halogenierung von gegebenenfalls substituierten Phthalsäuren oder deren Derivaten mit einem Halogenierungsmittel in rauchender Schwefelsäure in Gegenwart einer iodhaltigen Verbindung als Katalysator gefunden, das dadurch gekennzeichnet ist, daß die Halogenierung bei einer Temperatur von 100 bis 160°C erfolgt.

Bevorzugt erfolgt das erfindungsgemäße Verfahren bei einer Temperatur von 110 bis 150°C, insbesondere bei 120 bis 140°C.

Die eingesetzten Phthalsäuren bzw. deren Derivate können beispielsweise durch 1 bis 4 gleiche oder verschiedene Substituenten substituiert werden. Als beispielhafte Substituenten können genannt werden: Iod, Brom oder COOH.

Bevorzugt wird jedoch die unsubstituierte Phthalsäure bzw. das unsubstituierte Phthalsäureanhydrid eingesetzt. Unter Derivaten werden vorzugsweise Anhydride, Ester oder Salze verstanden. Ganz besonders bevorzugt sind die Anhydride. Die erfindungsgemäße Halogenierung ist beispielsweise eine Chlorierung, Bromierung oder Iodierung.

Bei der Verwendung von Halogenierungsmittel, die bei Reaktionstemperatur gasförmig oder leicht flüchtig sind, kann die Umsetzung unter erhöhtem Druck, vorzugsweise bis 5 bar, erfolgen.

Bei der Chlorierung wird als Chlorierungsmittel vorzugsweise gasförmiges Cl₂ verwendet, das im allgemeinen in die rauchende Schwefelsäure eingeleitet wird. Dabei kann bei Normaldruck oder erhöhtem Druck, vorzugsweise bis 5 bar, gearbeitet werden.

Bei der Bromierung wird bevorzugt Br₂ als Halogenierungsmittel verwendet.

Bei der Iodierung wird bevorzugt J₂ oder eine iodfreisetzende Verbindung eingesetzt. Besonders bevorzugt wird Iod eingesetzt. Als weitere Iodierungsmittel kommen beispielsweise in Frage: I₂, ICl, ICl₃, KI, NaI.

Die Menge des bei der Halogenierung einzusetzenden Halogenierungsmittel ist im allgemeinen abhängig davon, wieviel aromatische H-Atome oder gegebenenfalls sonstige Substituenten der eingesetzten Phthalsäure bzw. dessen Derivat durch Halogenatome ersetzt werden sollen. Für den Austausch von nur einem H-Atom bzw. Substituent, wobei als Substituent insbesondere ein Halogenatom verstanden wird, werden vorzugsweise 1 bis 1,5 mol des Halogenierungsmittels, insbesondere 1 bis 1,2 mol pro Mol der einzusetzenden Phthalsäure bzw. dessen Derivat verwendet. Für den Austausch von 2 H-Atomen bzw. Substituenten werden vorzugsweise 2 bis 3, insbesondere von 2 bis 2,4 mol, des jeweiligen Halogenierungsmittels pro Mol der eingesetzten Phthalsäure bzw. deren Derivat verwendet.

Für den Austausch von 3 H-Atomen bzw. Substituenten werden vorzugsweise 3 bis 4,5, insbesondere von 3 bis 3,6 mol, des jeweiligen Halogenierungsmittels pro Mol der eingesetzten Phthalsäure bzw. dessen Derivat verwendet.

Für den Austausch von 4 H-Atomen bzw. Substituenten werden vorzugsweise 4 bis 6, insbesondere von 4 bis 4,8 mol, des jeweiligen Halogenierungsmittels pro Mol der eingesetzten Phthalsäure bzw. dessen Derivat verwendet.

Besonders bevorzugt wird die Halogenierung des erfindungsgemäßen Verfahrens mit Cl₂ oder Br₂ als Halogenierungsmittel durchgeführt, wobei die Menge des Halogenierungsmittels insbesondere 4 bis 4,8 mol pro Mol des eingesetzten Phthalsäureanhydrids beträgt.

Als rauchende Schwefelsäure wird im erfindungsgemäßen Verfahren vorzugsweise eine solche mit einem SO₃-Gehalt von 5 bis 65 Gew.-%, insbesondere 15 bis 65 Gew.-%, eingesetzt.

Die erfindungsgemäß verwendeten Katalysatoren, insbesondere für die Chlorierung, können beispielsweise Iod, eine iodhaltige Interhalogenverbindung wie beispielsweise JCl₃, ICl oder aromatische Iodverbindungen, wie iodierte Toluole, iodierte Benzoesäuren oder insbesondere iodierte Phthalsäuren oder deren Derivate oder Gemische davon bedeuten. Der Einsatz iodierter Phthalsäuren besitzt den Vorteil, beispielsweise gegenüber der Verwendung von Iod, Iodid oder Iodchlor, daß die iodierten Phthalsäuren nicht flüchtig sind bzw. keine katalytisch wirksamen flüchtigen Verbindungen bilden und daher unter den Reaktionsbedingungen nicht aus dem Reaktionsgemisch entweichen können. Im Falle, daß iodierte Phthalsäuren oder deren Derivate als Katalystor, vorzugsweise bei der Chlorierung verwendet werden, wird als zu halogenierende Phthalsäure bzw. Phthalsäurederivat (Edukt) vorzugsweise eine solche eingesetzt, die mit dem eingesetzten Katalysator bis auf die Iodatome bzw. das Iodatom identisch ist, insbesondere mit dem Katalysator völlig identisch ist. In dem Fall besitzt das Edukt anstelle des Iodatoms oder der Iodatome, vorzugsweise H und/oder Halogen, insbesondere H, Cl oder Br. Ganz besonders bevorzugt entspricht die iodhaltige Verbindung Tetraiodphthalsäure oder insbesondere Tetraiodphthalsäureanhydrid. Das als Katalysator eingesetzte Iod bzw. die iodhaltige Verbindung wird im allgemeinen in katalytischen Mengen verwendet, vorzugsweise in einer Menge von 1 bis 10 Gew.-%, bezogen auf die eingesetzte Phthalsäure oder deren Derivat.

Die als Katalysator bevorzugt verwendete Tetraiodphthalsäure, insbesondere ihr Anhydrid, ist aus CH-50 177 bereits bekannt und kann beispielsweise durch Umsetzung von Iod mit Phthalsäuren oder deren Derivaten, insbesondere ihren Anhydriden, in rauchender Schwefelsäure bei einer Temperatur von 100 bis 180°C, hergestellt werden, wobei die Menge an Iod im allgemeinen 4 bis 8 Mol pro Mol eingesetzter Phthalsäure bzw. deren Derivat beträgt. Dabei besitzt die rauchende Schwefelsäure vorzugsweise ein SO₃-Gehalt von 60 bis 70 Gew.-%. Die hergestellte Tetraiodphthalsäure bzw. deren Anhydrid kann beispielsweise, wie in CH-50 177 beschrieben, isoliert und dem erfindungsgemäßen Verfahren als Katalysator zugesetzt werden. Vorzugsweise erfolgt jedoch keine Zwischenisolierung der Tetrachlorphthalsäure bzw. deren Anhydrid und die Reaktion wird nach Zusatz an einzusetzender Phthalsäure bzw. deren Anhydrid nach dem erfindungsgemäßen Verfahren fortgeführt.

Sofern die zu halogenierende gegebenenfalls substituierte Phthalsäure oder deren Derivat bei dem erfindungsgemäßen Verfahren zur Herstellung chlorierter oder bromierter Phthalsäuren eine iodierte Phthalsäure oder deren Derivat ist, so wird als Katalysator vorzugsweise ebenfalls diese Verbindung verwendet.

Das bevorzugte Gewichtsverhältnis von H₂SO₄ zu eingesetzter Phthalsäure bzw. -derivat beträgt 1:3 bis 1:7.

Nach dem erfindungsgemäßen Verfahren können auch halogenierte Phthalsäuren hergestellt werden, die unterschiedliche Halogenatome tragen. So können beispielsweise Mono-, Di- bzw. Trichlorphthalsäuren mit Iod oder Brom umgesetzt werden und liefern dabei die entsprechenden Iod-chlor- bzw. Brom-chlor-substituierten Phthalsäuren.

Nach Beendigung des erfindungsgemäßen Verfahrens, was beispielsweise mittels GC oder HPLC bestimmt wird, wird im allgemeinen so aufgearbeitet, daß man die Reaktionsmischung vorsichtig mit Wasser verdünnt und abfiltriert. Das Filtrat kann aufgrund seines geringen Glührückstandes wieder zu Oleum aufgearbeitet werden.

Die nach dem erfindungsgemäßen Verfahren hergestellten halogenierten Phthalsäuren bzw. deren Derivate zeichnen sich durch einen besonders geringen Anteil an Nebenprodukten aus.

So ist beispielsweise der Anteil an Iodtrichlorphthalsäure bzw. -anhydrid bei der Chlorierung gemäß dem erfindungsgemäßen Verfahren kleiner 1 %. Auch der Anteil an den Verunreinigungen ist mit dem erfindungsgemäßen Verfahren deutlich verringert worden. Damit ergeben sich Reinheiten der so erhältlichen halogenierten Phthalsäuren von vorzugsweise > 99 %.

Die Erfindung betrifft weiterhin die Verwendung der nach dem erfindungsgemäßen Verfahren hergestellten halogenierten Phthalsäuren bzw. -derivate, inbesonderen -anhydride als Zwischenprodukte für die Herstellung von Farbstoffen, Pigmenten, Fungiziden, Polyester- und Epoxidharzen mit flammhemmenden Eigenschaften sowie als flammhemmende Zusätze in Anstrichmitteln.

Die Erfindung betrifft auch Farbstoffe die unter Verwendung so hergestellter halogenierter Phthalsäuren oder deren Derivate hergestellt werden, wobei diese insbesondere in an sich bekannter Weise durch Kondensation der erfindungsgemäß hergestellten halogenierten Phthalsäuren oder deren Derivate mit aromatischen ortho- oder peri-Diaminen erhalten werden. Weiterhin betrifft die Erfindung Kunststoffe, gefärbt mit wenigstens einem erfindungsgemäßen Farbstoff. Die Kunststoffe, worunter insbesondere Thermoplaste, beispielsweise Vinylpolymere, Celluloseester, Polyester und Polyamide, insbesondere Polystyrol, Styrol-Acrylnitril-Copolymere, Styrol-Butadien-Copolymere, Styrol-Butadien-Acrylnitril-Terpolymere, Polymethylmethacrylat u.a. verstanden werden, werden vorzugsweise in der Masse gefärbt. Unter Massefärben wird insbesondere ein Verfahren verstanden, bei dem der Farbstoff in die geschmolzene Kunststoffmasse eingearbeitet wird, z.B. unter Zuhilfenahme eines Extruders oder bei dem der Farbstoff bereits den Ausgangskomponenten zur Herstellung des Kunststoffes, z.B. Monomeren vor der Polymerisation zugesetzt wird.

### Beispiel 1

In eine Vorlage aus 590 g 20 %igem Oleum, 148 g (1 mol) Phthalsäureanhydrid und 8 g (0,05 mol) Kaliumiodid wurden 312 g (4,4 mol) Chlor innerhalb von 15 Stunden bei einer Temperatur zwischen 120 und 130°C eingeleitet. Nach Beendigung der Reaktion wurde die Reaktionsmischung abgekühlt und anschließend 360 ml Wasser langsam zugetropft, so daß die Temperatur nicht über 50°C anstieg. Das erhaltene Rohprodukt wurde abfiltriert, mit Wasser gewaschen und getrocknet. Es wurden 94 % der Theorie Tetrachlorphthalsäureanhydrid, bezogen auf eingesetztes Phthalsäureanhydrid, erhalten, mit einer Reinheit von > 99 %.

### Beispiel 2

Es wurde wie in Beispiel 1 verfahren, jedoch wurden 6 g (0,05 mol) Iod anstatt Kaliumiodid eingetragen und die Chloreinleitung erfolgte über 16 Stunden bei einer Temperatur von 130°C. Während der Reaktion fiel Tetrachlorphthalsäureanhydrid in Form von langen Nadeln aus. Das erhaltene Tetrachlorphthalsäureanhydrid wurde in einer Ausbeute von 95 % der Theorie, bezogen auf eingesetztes Phthalsäureanhydrid mit einer Reinheit von > 99 % erhalten.

### Beispiel 3

Es wurde wie in Beispiel 1 verfahren, jedoch wurde als Katalysator 65 g (0,1 mol) Tetraiodphthalsäureanhydrid eingesetzt und das Chlor über 15 Stunden bei einer Temperatur von 120°C eingeleitet. Im Laufe der Reaktion fiel das entstandene Tetrachlorphthalsäureanhydrid in Form von langen Nadeln aus. Die Ausbeute betrug 97 % der Theorie, bezogen auf eingesetztes Phthalsäureahydrid. Die Reinheit beträgt > 99 %.

## Patentansprüche

1. Verfahren zur Herstellung halogenierter Phthalsäuren bzw. deren Derivate durch Halogenierung von gegebenenfalls substituierten Phthalsäuren bzw. deren Derivaten mit einem Halogenierungsmittel in rauchender Schwefelsäure in Gegenwart einer iodhaltigen Verbindung als Katalysator, dadurch gekennzeichnet, daß die Halogenierung bei einer Temperatur von 100 bis 160°C erfolgt.

2. Verfahren gemäß Anspruch 1, dadurch gekennzeichnet, daß die Halogenierung bei einer Tempeatur von 110 - 150, insbesondere 120 - 140°C erfolgt.

3. Verfahren gemäß Anspruch 1, dadurch gekennzeichnet, daß die eingesetzten Phthalsäuren bzw. deren Derivate gegebenenfalls durch 1 bis 4 gleiche oder verschiedene Substituenten aus der Reihe Iod, Brom oder COOH substituiert sind.

4. Verfahren gemäß Anspruch 1, dadurch gekennzeichnet, daß die eingesetzten Phthalsäuren bzw. deren Derivate unsubstituiert sind.

5. Verfahren gemäß Anspruch 1, dadurch gekennzeichnet, daß als Phthalsäurederivate Anhydride eingesetzt werden.

6. Verfahren gemäß Anspruch 1, dadurch gekennzeichnet, daß die rauchende Schwefelsäure 5 bis 65, insbesondere 15 bis 65 Gew.-% SO₃ enthält.

7. Verfahren gemäß Anspruch 1, dadurch gekennzeichnet, daß die Halogenierung mit Br₂ oder Cl₂ als Halogenierungsmittel erfolgt.

8. Verfahren gemäß Anspruch 1, dadurch gekennzeichnet, daß der Katalysator in einer Menge von 1 bis 10 Gew.-%, bezogen auf die eingesetzte Phthalsäure oder deren Derivate, eingesetzt wird.

9. Verfahren gemäß Anspruch 1, dadurch gekennzeichnet, daß als Katalysator Iod, Kaliumiodid, Natriumiodid, JCl, JCl₃ oder eine aromatische Iodverbindung, insbesondere eine iodierte Phthalsäure oder deren Derivat oder Gemische davon eingesetzt werden.

10. Verfahren gemäß Anspruch 1, dadurch gekennzeichnet, daß als Katalysator Tetraiodphthalsäure oder deren Anhydrid verwendet wird.

11. Verwendung der nach dem Verfahren gemäß Anspruch 1 hergestellten halogenierten Phthalsäuren bzw. deren Derivate als Zwischenprodukte zur Herstellung von Farbstoffen, Pigmenten, Fungiziden, Polyester- und Epoxidharzen mit flammhemmenden Eigenschaften sowie als flammhemmende Zusätze in Anstrichmitteln.

12. Farbstoffe, hergestellt unter Verwendung der nach einem Verfahren der Ansprüche 1 - 10 hergestellten halogenierten Phthalsäuren oder deren Derivaten.

13. Kunststoffe gefärbt mit einem Farbstoff gemäß Anspruch 11.
